# EUROPEAN PATENT APPLICATION

(11) **EP 4 245 768 A1**
(43) Date of publication of application: **20.09.2023**
(21) Application number: 21891204.6
(22) Date of filing: 12.11.2021
(51) Int. Cl.: C07K 14/55, C12N 15/26, A61K 38/20, A61K 47/00

(54) **PHARMACEUTICAL COMPOSITION COMPRISING HUMAN INTERLEUKIN 2 VARIANT OR DERIVATIVE THEREOF AND USE THEREOF**

(30) Priority: 13.11.2020 CN 202011269123; 02.06.2021 CN 202110614713
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN); Shanghai Shengdi Pharmaceutical Co., Ltd, Shanghai 201210 (CN)
(72) Inventor: YE, Linmao, Lianyungang, Jiangsu 222047 (CN); FAN, Yingfang, Lianyungang, Jiangsu 222047 (CN); SUN, Yanyan, Lianyungang, Jiangsu 222047 (CN); YU, Shuxiang, Lianyungang, Jiangsu 222047 (CN); CHEN, Hao, Lianyungang, Jiangsu 222047 (CN)
(74) Representative: Regimbeau
(86) International application number: PCT/CN2021/130246
(87) International publication number: WO 2022/100684

(57) **Abstract**

Provided are a pharmaceutical composition comprising a human interleukin 2 (IL-2) variant or derivative thereof and use thereof. In particular, provided are a pharmaceutical composition including a human interleukin 2 variant or derivative thereof and a pharmaceutically acceptable excipient. The pharmaceutical composition has improved high temperature, freezing and thawing, room-temperature stability, and appearance formulation reproducibility.

## Description

### TECHNICAL FIELD

The present disclosure relates to the field of pharmaceutical preparations and particularly to a pharmaceutical composition comprising a human interleukin 2 variant or a derivative thereof.

### BACKGROUND

Human interleukin-2 (IL-2), also known as T cell growth factor (TCGF), consists of 133 amino acids and has a molecular weight of about 15 kD, and its coding gene is located in chromosome 4 (4q27) and comprises a sequence of 7 kb in total. In 1976 and 1977, Doris Morgan, Francis Ruscetti, Robert Gallo and Steven Gillis, Kendal Smith, et al., separately found that activated T cell culture medium could promote T cell proliferation. Thereafter, the stimulating factor in the culture medium was purified and identified as a single protein, i.e., IL-2. IL-2 has an anti-tumor effect due to its ability to expand the lymphocyte population and enhance the effector function of these cells. IL-2 immunotherapy has become a treatment option for some patients with metastatic cancer. High doses of IL-2 have now been approved for the treatment of metastatic renal cell carcinoma and malignant melanoma.

Several pharmaceutical companies are developing IL-2 variants in some countries; related patent applications include WO2012062228, CN201280017730.1, US8906356, US9732134, US7371371, US7514073, US8124066, US7803361, WO2016014428, and the like. For example, WO2020125743 relates to a class of novel IL-2 variants and derivatives thereof, which have higher stability and, as immunotherapeutics, have improved properties.

The IL-2 variants and derivatives thereof are protein drugs, which are easily degraded when orally taken; they have the properties of being thermally unstable and easy to hydrolyze. It is of great significance to obtain stable preparations with good appearances. US4604377 describes a lyophilized IL-2 preparation, which comprises mannitol as a stabilizer and sodium dodecyl sulfate (SDS) or sodium deoxycholate sulfate as a solubilizer. US5417970 describes a lyophilized IL-2 preparation, which comprises hydrolyzed gelatin or human serum albumin and alanine. However, SDS may bind proteins and thus be difficult to remove, which is unfavorable for renaturation; the market for serum albumin is big, the price of serum albumin is high, and the process of serum albumin production is complicated. ZL01814445.4 discloses a stable IL-2 preparation containing histidine, sucrose and glycine and reveals that Tween 80 promotes the formation of soluble IL-2 aggregates and there is a noticeable amount of soluble aggregates as early as before lyophilization. Wang et al. (Wei W et al. Dual effects of Tween 80 on protein stability[J]. Int J Pharm, 2008, 347(1-2):31-38) also found that although Tween 80 significantly inhibits shaking-induced aggregation of IL-2 mutant proteins, Tween 80 affects the storage stability of IL-2 in a way associated with temperature: in the case of storage at 5 °C for 22 months, the presence of Tween 80 results in barely detectable aggregation; during 2 months of storage at 40 °C, addition of 0.1% Tween 80 significantly accelerates the aggregation of IL-2 mutant proteins.

In addition, appearance is one of the important markers of the quality of a lyophilized article of manufacture. An acceptable lyophilized article of manufacture should be a porous solid with a uniform color and a fine texture. In mass production, cakes of a lyophilized article of manufacture sometimes have unacceptable appearances due to contraction, cracks, and the like, which lead to heavy financial losses; the appearance of the lyophilized article of manufacture is affected by excipient ingredients.

In the present disclosure, on the basis of the pharmaceutical characteristics and dosage form characteristics of IL-2, a large amount of screening is done with respect to pH, buffer, excipient, surfactant, etc. according to indicators such as appearance, RP-HPLC\SE-HPLC\IE-HPLC purities and stability, and a pharmaceutical composition comprising IL-2 is obtained. The pharmaceutical composition has improved high-temperature, freeze-thaw and room-temperature stabilities and appearance preparation reproducibility. The present disclosure provides a product with better performance for practical production and clinical application.

### SUMMARY

The present disclosure provides a pharmaceutical composition comprising IL-2, mannitol, and trehalose.

In some embodiments, the IL-2 in the pharmaceutical composition described above is at a concentration of about 0.1 mg/mL to 100 mg/mL; in some specific embodiments, IL-2 is at a concentration of about 1 mg/mL to about 50 mg/mL, about 1 mg/mL to about 10 mg/mL, or about 2 mg/mL to about 5 mg/mL.

In some embodiments, IL-2 is at a concentration of about 0.1 mg/mL, 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, about 1.0 mg/mL, about 1.1 mg/mL, about 1.2 mg/mL, about 1.3 mg/mL, about 1.4 mg/mL, about 1.5 mg/mL, about 1.6 mg/mL, about 1.7 mg/mL, about 1.8 mg/mL, about 1.9 mg/mL, about 2.0 mg/mL, about 2.1 mg/mL, about 2.2 mg/mL, about 2.3 mg/mL, about 2.4 mg/mL, about 2.5 mg/mL, about 2.6 mg/mL, about 2.7 mg/mL, about 2.8 mg/mL, about 2.9 mg/mL, about 3.0 mg/mL, about 3.1 mg/mL, about 3.2 mg/mL, about 3.3 mg/mL, about 3.4 mg/mL, about 3.5 mg/mL, about 3.6 mg/mL, about 3.7 mg/mL, about 3.8 mg/mL, about 3.9 mg/mL, about 4.0 mg/mL, about 4.1 mg/mL, about 4.2 mg/mL, about 4.3 mg/mL, about 4.4 mg/mL, about 4.5 mg/mL, about 4.6 mg/mL, about 4.7 mg/mL, about 4.8 mg/mL, about 4.9 mg/mL, about 5.0 mg/mL, about 6.0 mg/mL, about 7.0 mg/mL, about 8.0 mg/mL, about 9.0 mg/mL, about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, or about 100 mg/mL.

In some specific embodiments, IL-2 is at a concentration of about 2 mg/mL.

### Mannitol and Trehalose

In some embodiments, the pharmaceutical composition of the present disclosure comprises a sugar. The "sugar" includes general compositions (CH₂O)ₙ and derivatives thereof, including monosaccharides, disaccharides, trisaccharides, polysaccharides, sugar alcohols, reducing sugars, non-reducing sugars, and the like. The sugar may be selected from the group consisting of glucose, sucrose, trehalose, lactose, fructose, maltose, dextran, glycerin, erythritol, glycerol, arabitol, sylitol, sorbitol, mannitol, mellibiose, melezitose, raffinose, mannotriose, stachyose, maltose, lactulose, maltulose, glucitol, maltitol, lactitol, iso-maltulose, and the like.

In some embodiments, the pharmaceutical composition of the present disclosure comprises mannitol and trehalose.

In some embodiments, mannitol is at a concentration of about 5 mg/mL to about 100 mg/mL. In some specific embodiments, mannitol is at a concentration of about 10 mg/mL to about 50 mg/mL, about 15 mg/mL to 40 mg/mL, about 20 mg/mL to 35 mg/mL, about 25 mg/mL to 40 mg/mL, or about 25 mg/mL to 35 mg/mL, for example, about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, or about 100 mg/mL, or about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL, about 20 mg/mL, about 21 mg/mL, about 22 mg/mL, about 23 mg/mL, about 24 mg/mL, about 25 mg/mL, about 26 mg/mL, about 27 mg/mL, about 28 mg/mL, about 29 mg/mL, about 30 mg/mL, about 31 mg/mL, about 32 mg/mL, about 33 mg/mL, about 34 mg/mL, about 35 mg/mL, about 36 mg/mL, about 37 mg/mL, about 38 mg/mL, about 39 mg/mL, about 40 mg/mL, about 41 mg/mL, about 42 mg/mL, about 43 mg/mL, about 44 mg/mL, about 45 mg/mL, about 46 mg/mL, about 47 mg/mL, about 48 mg/mL, about 49 mg/mL, or about 50 mg/mL.

In some specific embodiments, mannitol is at a concentration of about 30 mg/mL.

In some embodiments, the trehalose in the pharmaceutical composition of the present disclosure includes trehalose or hydrates thereof; in some specific embodiments, the trehalose is trehalose dihydrate.

In some embodiments, the amount of trehalose is based on trehalose dihydrate; with a concentration of about 10 mg/mL to about 100 mg/mL, about 20 mg/mL to about 80 mg/mL, about 30 mg/mL to about 70 mg/mL, about 30 mg/mL to about 50 mg/mL, about 30 mg/mL to about 45 mg/mL, or about 35 mg/mL to about 40 mg/mL, for example, about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, or about 100 mg/mL.

In some embodiments, the amount of trehalose is based on trehalose dihydrate; with a concentration of about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL, about 20 mg/mL, about 21 mg/mL, about 22 mg/mL, about 23 mg/mL, about 24 mg/mL, about 25 mg/mL, about 26 mg/mL, about 27 mg/mL, about 28 mg/mL, about 29 mg/mL, about 30 mg/mL, about 31 mg/mL, about 32 mg/mL, about 33 mg/mL, about 34 mg/mL, about 35 mg/mL, about 36 mg/mL, about 37 mg/mL, about 38 mg/mL, about 39 mg/mL, about 40 mg/mL, about 41 mg/mL, about 42 mg/mL, about 43 mg/mL, about 44 mg/mL, about 45 mg/mL, about 46 mg/mL, about 47 mg/mL, about 48 mg/mL, about 49 mg/mL, about 50 mg/mL, about 51 mg/mL, about 52 mg/mL, about 52.5 mg/mL, about 53 mg/mL, about 54 mg/mL, about 55 mg/mL, about 56 mg/mL, about 57 mg/mL, about 58 mg/mL, about 59 mg/mL, about 60 mg/mL, about 61 mg/mL, about 62 mg/mL, about 63 mg/mL, about 64 mg/mL, about 65 mg/mL, about 66 mg/mL, about 67 mg/mL, about 68 mg/mL, about 69 mg/mL, about 70 mg/mL, about 71 mg/mL, about 72 mg/mL, about 73 mg/mL, about 74 mg/mL, about 75 mg/mL, about 76 mg/mL, about 77 mg/mL, about 78 mg/mL, about 79 mg/mL, or about 80 mg/mL.

In some specific embodiments, the amount of trehalose is based on trehalose dihydrate; with a concentration of about 40 mg/mL.

In some embodiments, mannitol and trehalose (based on trehalose dihydrate) are in a ratio of 1:10 to 10:1, 1:7 to 7:1, 1:6 to 6:1, 1:5 to 5:1, 1:4 to 4:1, 1:3 to 3:1, 1:2 to 2:1, 1:7 to 3:4, 1:7 to 6:7, 1:7 to 1:1, 1:6 to 3:4, 1:6 to 6:7, 1:6 to 1:1, 1:5 to 3:4, 1:5 to 6:7, 1:5 to 1:1, 1:4 to 3:4, 1:4 to 6:7, 1:4 to 1:1, 1:3 to 3:4, 1:3 to 6:7, 1:3 to 1:1, 1:2 to 3:4, 1:2 to 6:7, 1:2 to 1:1, or 6:8 to 6:7, for example, 1:10, 1:9, 1:8, 1:7, 1:6, 1:5, 1:4, 1:3, 1:2, 1:1, 2:9, 2:8, 2:7, 2:6, 2:5, 2:4, 2:3, 2:2, 2:1, 3:9, 3:8, 3:7, 3:6, 3:5, 3:4, 3:3, 3:2, 3:1, 4:9, 4:8, 4:7, 4:6, 4:5, 4:4, 4:3, 4:2, 4:1, 5:9, 5:8, 5:7, 5:6, 5:5, 5:4, 5:3, 5:2, 5:1, 6:9, 6:8, 6:7, 6:6, 6:5, 6:4, 6:3, 6:2, 6:1, 7:9, 7:8, 7:7, 7:6, 7:5, 7:4, 7:3, 7:2, 7:1, 8:9, 8:8, 8:7, 8:6, 8:5, 8:4, 8:3, 8:2, 8:1, 9:9, 9:8, 9:7, 9:6, 9:5, 9:4, 9:3, 9:2, 9:1, 10:1, 10:9, 10:8, 10:7, 10:6, 10:5, 10:4, 10:3, 10:2, or 10:1.

The ratio of mannitol to trehalose described above is a mass ratio and can actually be calculated by conversion of the concentrations of mannitol and trehalose, and the method of such conversion is well known in the art.

Similarly, the mass ratio of IL-2 to mannitol or trehalose in the pharmaceutical composition of the present disclosure may also be calculated by conversion of their concentrations.

In some embodiments, IL-2 and mannitol are in a mass ratio ranging from 1:1 to 1:50; for example, the mass ratio is 1:1, 1:2, 1:3, 1:4, 1:5, 1:6, 1:7, 1:8, 1:9, 1:10, 1:15, 1:20, 1:25, 1:30, 1:35, 1:40, 1:45, or 1:50.

In some embodiments, IL-2 and trehalose are in a mass ratio ranging from 4:50 to 4:150; for example, the mass ratio is 4:50, 4:60, 4:70, 4:80, 4:90, 4:105, 4:120, or 4:140.

### Surfactant

In some embodiments, the pharmaceutical composition of the present disclosure further comprises a surfactant. The surfactant may be selected from the group consisting of polysorbate 20, polysorbate 80, poloxamer, Triton, sodium dodecyl sulfonate, sodium lauryl sulfonate, sodium octyl glycoside, lauryl-, myristyl-, linoleyl- or stearyl-sulfobetaine, lauryl-, myristyl-, linoleyl- or stearyl-sarcosine, linoleyl-, myristyl- or cetyl-betaine, lauramido propyl-, cocaramide propyl-, linoleinamide propyl-, myristylamide propyl-, palmitamide propyl- or isostearamide propyl-betaine, myristylamide propyl-, palmitamide propyl- or isostearamide propyl-dimethylamine, sodium methyl cocoyl, sodium methyl oleyl taurate, polyethylene glycol, polypropylene glycol, copolymer of ethylene and propylene glycol, and the like.

In some embodiments, the pharmaceutical composition of the present disclosure comprises a polysorbate (e.g., polysorbate 20, polysorbate 40, polysorbate 60, or polysorbate 80). In some specific embodiments, the pharmaceutical composition of the present disclosure comprises polysorbate 80. The present disclosure reveals that polysorbates may act as stabilizers by reducing aggregation of protein drugs. Polysorbate (e.g., polysorbate 80) is at a concentration of about 0.01 mg/mL to about 0.2 mg/mL, about 0.02 mg/mL to about 0.15 mg/mL, about 0.02 mg/mL to about 0.1 mg/mL, about 0.03 mg/mL to about 0.1 mg/mL, about 0.04 mg/mL to about 0.1 mg/mL, 0.02 mg/mL to about 0.05 mg/mL, or about 0.05 mg/mL to about 0.1 mg/mL, for example, about 0.01 mg/mL, about 0.02 mg/mL, about 0.03 mg/mL, about 0.04 mg/mL, about 0.05 mg/mL, about 0.06 mg/mL, about 0.07 mg/mL, about 0.08 mg/mL, about 0.09 mg/mL, about 0.1 mg/mL, about 0.15 mg/mL, or about 0.2 mg/mL.

In some specific embodiments, polysorbate (e.g., polysorbate 80) is at a concentration of about 0.05 mg/mL.

### Buffer System

In some embodiments, the pharmaceutical composition of the present disclosure further comprises a buffer. The buffering salt includes common hydrates of salts, such as citrate buffers, acetate buffers, histidine salt buffers, phosphate buffers, carbonate buffers, and succinate buffers.

In some embodiments, the buffer is a histidine salt buffer system, wherein the histidine salt buffer is selected from the group consisting of a histidine-hydrochloric acid buffer, a histidine-acetate buffer, a histidine-phosphate buffer, a histidine-sulfate buffer, and the like.

In some specific embodiments, the buffer is a histidine-hydrochloric acid buffer.

In some embodiments, the buffer in the pharmaceutical composition has a concentration of about 2 mM to about 50 mM, about 5 mM to about 40 mM, about 5 mM to about 30 mM, about 5 mM to about 20 mM, about 5 mM to about 15 mM, or about 5 mM to about 10 mM, for example, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 25 mM, about 30 mM, about 40 mM, or about 50 mM.

In some specific embodiments, the buffer in the pharmaceutical composition has a concentration of about 10 mM.

In some embodiments, the buffer in the pharmaceutical composition has a pH of about 4.5 to about 6.0, about 5.0 to about 6.0, about 5.0 to about 5.6, or about 5.3 to about 5.5, for example, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, or about 6.0.

In some specific embodiments, the buffer in the pharmaceutical composition has a pH of about 5.3.

### Solvent

In some embodiments, the pharmaceutical composition of the present disclosure further comprises a solvent.

The solvent in the pharmaceutical composition is selected from, but is not limited to, a non-toxic physiologically acceptable liquid carrier, such as normal saline, water for injection, or a glucose solution (e.g. 5% glucose injection or glucose-sodium chloride injection).

In addition, the present disclosure also provides a pharmaceutical composition comprising IL-2, including but not limited to:
(1) a pharmaceutical composition comprising IL-2, about 30 mg/mL mannitol, about 40 mg/mL trehalose, and about 0.05 mg/mL polysorbate, and having a pH of about 5.3, wherein the IL-2 is at a concentration of about 1 mg/mL, about 2 mg/mL, about 5 mg/mL, about 10 mg/mL, or about 100 mg/mL;
(2) a pharmaceutical composition comprising IL-2, about 30 mg/mL mannitol, about 40 mg/mL trehalose, and 0.1 mg/mL polysorbate, and having a pH of about 5.3, wherein the IL-2 is at a concentration of about 1 mg/mL, about 2 mg/mL, about 5 mg/mL, about 10 mg/mL, or about 100 mg/mL;
(3) a pharmaceutical composition comprising about 2 mg/mL IL-2, mannitol, about 40 mg/mL trehalose, and about 0.1 mg/mL polysorbate, and having a pH of about 5.3, wherein the mannitol is at a concentration of about 10 mg/mL, about 20 mg/mL, about 30 mg/mL, about 35 mg/mL, or about 50 mg/mL;
(4) a pharmaceutical composition comprising about 2 mg/mL IL-2, mannitol, about 40 mg/mL trehalose, and about 0.05 mg/mL polysorbate, and having a pH of about 5.3, wherein the mannitol is at a concentration of about 10 mg/mL, about 20 mg/mL, about 30 mg/mL, about 35 mg/mL, or about 50 mg/mL;
(5) a pharmaceutical composition comprising about 2 mg/mL IL-2, about 30 mg/mL mannitol, trehalose, and about 0.1 mg/mL polysorbate, and having a pH of about 5.3, wherein the trehalose is at a concentration of about 10 mg/mL, about 20 mg/mL, about 30 mg/mL, about 35 mg/mL, about 40 mg/mL, or about 50 mg/mL; and
(6) a pharmaceutical composition comprising about 2 mg/mL IL-2, about 30 mg/mL mannitol, trehalose, and about 0.05 mg/mL polysorbate, and having a pH of about 5.3, wherein the trehalose is at a concentration of about 10 mg/mL, about 20 mg/mL, about 30 mg/mL, about 35 mg/mL, about 40 mg/mL, or about 50 mg/mL.

In some specific embodiments, the above pharmaceutical compositions (1) to (6) further comprise about 10 mM histidine salt buffer, e.g., histidine-hydrochloric acid buffer.

In some specific embodiments, the IL-2 disclosed in the above pharmaceutical compositions (1) to (6) is an IL-2 variant or a derivative thereof, and the IL-2 variant or the derivative thereof contains site mutations N26Q, N29S and N88R and may or may not contain site mutation C125A; for example, the IL-2 variant or the derivative thereof comprises an amino acid sequence set forth in SEQ ID NO: 2.

In some embodiments, the IL-2 is an IL-2 variant or derivative. The IL-2 includes IL-2 variants or derivatives thereof, and in alternative embodiments, the IL-2 variant or the derivative thereof in the aforementioned pharmaceutical composition contains one or more amino acid mutations at positions 11, 26, 27, 29, 30, 31, 32, 33, 34, 35, 36, 37, 38, 39, 40, 41, 42, 43, 44, 70, 71, 72, 78, 82, 88, 125 and 132 of wild-type human IL-2. In the present disclosure, mutations are expressed as abc, where a denotes the amino acid type before mutation, b denotes the mutation position, and c denotes the amino acid type after mutation. For example, N26S indicates that asparagine (N) is mutated into serine (S) at position 26; N26 indicates that asparagine (N) at position 26 is mutated; 26S indicates that the amino acid at position 26 is mutated into serine (S).

Specifically, the IL-2 variant or the derivative thereof provided by the present disclosure contains one or more amino acid mutations or any combination thereof at the following positions: 26, 29, 30, 71, 11, 132, 70, 82, 27, and 78. In some embodiments, the amino acids (e.g., in wild-type human IL-2) before the mutations are: asparagine (N) at position 26, asparagine (N) at position 29, asparagine (N) at position 30, asparagine (N) at position 71, glutamine (Q) at position 11, leucine (L) at position 132, leucine (L) at position 70, proline (P) at position 82, glycine (G) at position 27, and phenylalanine (F) at position 78.

In some specific embodiments, the IL-2 variant or the derivative thereof contains a first class of mutations, wherein the first class of mutations is any one of (1) to (7) or any combination thereof:
(1) N26Q,
(2) N29S,
(3) N30S,
(4) N71Q,
(5) Q11C and L132C,
(6) L70C and P82C, and
(7) G27C and F78C.

In some specific embodiments, the IL-2 variant or the derivative thereof described above has higher stability, e.g., more deamination stability and/or thermostability; specifically, the first class of mutations provided by the present disclosure makes the IL-2 variant or the derivative thereof containing the mutations have higher stability than wild-type IL-2, including but not limited to higher deamination stability and/or thermostability.

In some specific embodiments, the IL-2 variant or the derivative thereof contains a second class of mutations, wherein the second class of mutations includes one or more amino acid mutations or any combination thereof at the following positions: 20. 88, and 126. In some embodiments, the amino acids (e.g., in wild-type human IL-2) before the mutations are: aspartic acid (D) at position 20, asparagine (N) at position 88, and glutamine (Q) at position 126. In some specific embodiments, the IL-2 variant or the derivative thereof further contains a second class of mutations, wherein the second class of mutations is selected from any one of the following mutations or any combination thereof: the mutation of N88 into A, R, E, L, F, G, I, M, S, Y, V, or D; the mutation of D20 into A, H, I, M, E, S, V, W, or Y, and the mutation of Q126 into N, L, P, F, G, I, M, R, S, T, Y, or V

In some specific embodiments, the second class of mutations is selected from any one of (8) to (10) or any combination thereof:
(8) N88R or N88G or N88I or N88D,
(9) D20H or D20Y, and
(10) Q126L.

The second class of mutations enables the proliferation-inducing and activating functions of IL-2 on Treg to be retained, but eliminates or reduces the proliferation-inducing and activating functions of IL-2 on effector cells (such as NK and T cells).

In some embodiments, the IL-2 variant or the derivative thereof contains both the first and second classes of mutations described above and, optionally, may or may not contain site mutation C125A, wherein the first class of mutations is selected from any one of (11) to (13):
(11) N26Q and N29S,
(12) N26Q, N29S and N71Q, and
(13) N26Q and N30S;
the second class of mutations is N88R or N88G or N88I or N88D.

In some embodiments, the IL-2 variant or the derivative thereof contains (14) mutations N26Q, N29S, and N88R.

The IL-2 variant or the derivative thereof described herein contains any combination of the mutation positions and mutation types in (1) to (13) and includes, but is not limited to, the IL-2 variants disclosed in WO2020125743A.

In some embodiments, the mutations described above occur relative to wild-type IL-2; the wild-type IL-2 comprises an amino acid sequence set forth in SEQ ID NO: 1. The positions of the mutations of the present disclosure are numbered according to the amino acid sequence set forth in SEQ ID NO: 1 with the amino acid A at position 2 as a start. Any of the IL-2 variants of the present disclosure comprises or does not comprise the methionine (M) at position 1 according to SEQ ID NO: 1.

In some embodiments, the IL-2 variant or the derivative thereof comprises an amino acid sequence selected from any one of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and SEQ ID NO: 12. The amino acids and corresponding nucleotide sequence numbers of the polypeptides are shown in Table 1 (the mutated amino acids are underlined):

**Table 1. Amino acid sequences and nucleic acid sequences of IL-2 variants**

| Mutation position | Amino acid sequences of wild-type human IL-2 and variants |
|---|---|
| Wild type | MAPTSSSTKKTQLQLEHLLLDLQMILNGINNYKNPKLTRMLTFKFY MPKKATELKHLQCLEEELKPLEEVLNLAQSKNFHLRPRDLISNINVI VLELKGSETTFMCEYADETATIVEFLNRWITFCQSIISTLT (SEQ ID NO: 1) |
| N26Q/N29S/N 88R | MAPTSSSTKKTQLQLEHLLLDLQMILQGISNYKNPKLTRMLTFKFY MPKKATELKHLQCLEEELKPLEEVLNLAQSKNFHLRPRDLISRINVI VLELKGSETTFMCEYADETATIVEFLNRWITFAQSIISTLT (SEQ ID NO: 2) |
| N26Q | MAPTSSSTKKTQLQLEHLLLDLQMILQGINNYKNPKLTRMLTFKFY MPKKATELKHLQCLEEELKPLEEVLNLAQSKNFHLRPRDLISNINVI VLELKGSETTFMCEYADETATIVEFLNRWITFAQSIISTLT (SEQ ID NO: 3) |
| N29S | MAPTSSSTKKTQLQLEHLLLDLQMILNGISNYKNPKLTRMLTFKFY MPKKATELKHLQCLEEELKPLEEVLNLAQSKNFHLRPRDLISNINVI VLELKGSETTFMCEYADETATIVEFLNRWITFAQSIISTLT (SEQ ID NO: 4) |
| Q11C/L132C | MAPTSSSTKKTCLQLEHLLLDLQMILNGINNYKNPKLTRMLTFKFY MPKKATELKHLQCLEEELKPLEEVLNLAQSKNFHLRPRDLISNINVI VLELKGSETTFMCEYADETATIVEFLNRWITFAQSIISTCT (SEQ ID NO: 5) |
| L70C/P82C | MAPTSSSTKKTQLQLEHLLLDLQMILNGINNYKNPKLTRMLTFKFY MPKKATELKHLQCLEEELKPLEEVCNLAQSKNFHLRCRDLISNINV IVLELKGSETTFMCEYADETATIVEFLNRWITFAQSIISTLT (SEQ ID NO: 6) |
| G27C/F78C | MAPTSSSTKKTQLQLEHLLLDLQMILNCINNYKNPKLTRMLTFKFY MPKKATELKHLQCLEEELKPLEEVLNLAQSKNCHLRPRDLISNINV IVLELKGSETTFMCEYADETATIVEFLNRWITFAQSIISTLT (SEQ ID NO: 7) |
| N26Q/N29S/N 88G | MAPTSSSTKKTQLQLEHLLLDLQMILQGISNYKNPKLTRMLTFKFY MPKKATELKHLQCLEEELKPLEEVLNLAQSKNFHLRPRDLISGINVI VLELKGSETTFMCEYADETATIVEFLNRWITFAQSIISTLT (SEQ ID NO: 8) |
| N26Q/N29S/N 881 | MAPTSSSTKKTQLQLEHLLLDLQMILQGISNYKNPKLTRMLTFKFY MPKKATELKHLQCLEEELKPLEEVLNLAQSKNFHLRPRDLISIINVI VLELKGSETTFMCEYADETATIVEFLNRWITFAQSIISTLT (SEQ ID NO: 9) |
| N26Q/N29S/N 88D | MAPTSSSTKKTQLQLEHLLLDLQMILQGISNYKNPKLTRMLTFKFY MPKKATELKHLQCLEEELKPLEEVLNLAQSKNFHLRPRDLISDINVI VLELKGSETTFMCEYADETATIVEFLNRWITFAQSIISTLT (SEQ ID NO: 10) |
| N26Q/N30S/N 88R | MAPTSSSTKKTQLQLEHLLLDLQMILQGINSYKNPKLTRMLTFKFY MPKKATELKHLQCLEEELKPLEEVLNLAQSKNFHLRPRDLISRINVI VLELKGSETTFMCEYADETATIVEFLNRWITFAQSIISTLT (SEQ ID NO: 11) |
| N26Q/N29S/N 71Q/N88R | MAPTSSSTKKTQLQLEHLLLDLQMILQGISNYKNPKLTRMLTFKFY MPKKATELKHLQCLEEELKPLEEVLQLAQSKNFHLRPRDLISRINVI VLELKGSETTFMCEYADETATIVEFLNRWITFAQSIISTLT (SEQ ID NO: 12) |

| | |
|---|---|
| (Note: The mutation positions described above are numbered according to the numbering for the mature human IL-2 protein set forth in SEQ ID NO: 1. The mature human IL-2 protein does not comprise amino acid M at position 1, so the numbering takes amino acid A at position 2 as a start. "/" indicates that the mutations are present simultaneously in the same IL-2 variant. All variants may or may not contain C125A. | |

To contain C125A is to avoid dimer formation.)

In some specific embodiments, the IL-2 variant or the derivative thereof comprises an amino acid sequence set forth in SEQ ID NO: 2.

In some specific embodiments, the IL-2 variant or the derivative thereof has a structure of formula I which has a relative molecular weight of about 36 kD, wherein the structure of formula I comprises an IL-2 variant amino acid sequence set forth in SEQ ID NO: 2. 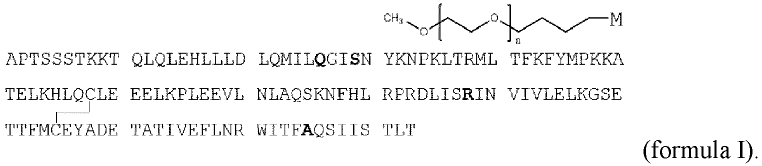 (Note: The positions in bold are positions of mutations relative to the wild type: N26Q, N29S, N88R, C125A. The linked Cys58-Cys105 indicates the position of a disulfide bond.)

In some specific embodiments, the structure of formula I is prepared from an IL-2 variant set forth in SEQ ID NO: 2 and a PEG molecule with a relative molecular weight of about 20 KD, and the total relative molecular weight of the structure of formula I is about 36 kD.

In some embodiments, the derivative of the IL-2 variant includes the full-length or partial proteins related to the IL-2 variant of the present disclosure or mutant proteins, functional derivatives, functional fragments, biologically active peptides, fusion proteins, isoforms or salts thereof obtained by further mutation on the basis of the IL-2 variant of the present disclosure, for example, fusion proteins comprising the IL-2 variant, the monomer or dimer or trimer or polymers of the IL-2 variant, various modified forms of the IL-2 variant (e.g., PEGylated, glycosylated, albumin-conjugated or -fused, Fc-fused or -conjugated, hydroxyethylated, and de-O-glycosylated), and homologs of the IL-2 variant in various species. The modifications to IL-2 do not result in adverse effects on the immunogenicity associated with treatment.

In some embodiments, the IL-2 variant or derivative is PEGylated (may be denoted by PEG-IL-2), e.g., mono- or di-PEGylated. The PEG-IL-2 variant or derivative comprises an SC-PEG linking group. In some other embodiments, the PEG-IL-2 variant or derivative comprises a methoxy-PEG-aldehyde (mPEG-ALD) linking group. In certain embodiments, the PEG portion has an average molecular weight of about 5 KD to about 50 KD, specifically about 5, about 10, about 11, about 12, about 13, about 14, about 15, about 16, about 17, about 18, about 19, about 20, about 21, about 22, about 23, about 24, about 25, about 30, about 35, about 40, about 45, or about 50 KD; or about 5 KD to about 40 KD, or about 10 KD to about 30 KD, or between about 10 KD and about 30 KD, or between about 15 KD and about 20 KD. In certain specific embodiments, the mPEG-ALD linking group comprises a PEG molecule having an average molecular weight selected from the group consisting of about 5 kDa, about 12 kDa and about 20 kDa (quality control standard: 20 ± 2 KDa). In certain embodiments, the aldehyde group of mPEG-ALD may be acetaldehyde, propionaldehyde, butyraldehyde, etc. In one embodiment, the IL-2 variant or the derivative thereof has a longer serum half-life than wild-type IL-2 or derivatives thereof.

When the IL-2 variant or the derivative thereof contains the second class of mutations, in some embodiments, the IL-2 variant or the derivative thereof can reduce the affinity of IL-2 for high-affinity receptors (IL-2Rα/β/γ) and moderate-affinity receptors (IL-2Rβ/γ), but the reductions in affinity for high-affinity receptors are greater than the reductions in affinity for moderate-affinity receptors. In some embodiments, the IL-2 variant or the derivative thereof can retain the proliferation-inducing and activating functions of IL-2 on Treg, but eliminate or reduce the proliferation-inducing and activating functions of IL-2 on effector cells (such as NK and T cells).

The IL-2 variants or the derivatives thereof and related sequences thereof and preparation methods therefor in WO2020/125743 are incorporated herein in their entirety.

### Lyophilized Preparation

The present disclosure also provides a method for preparing a lyophilized preparation of a pharmaceutical composition comprising IL-2, which comprises the step of lyophilizing the aforementioned pharmaceutical composition.

The present disclosure also provides a lyophilized preparation of a pharmaceutical composition comprising IL-2, which is obtained by lyophilizing any one of the aforementioned IL-2 pharmaceutical compositions. The process for preparing a lyophilized preparation comprises the following steps:
① preparing a histidine salt buffer and preparing an excipient mother liquor;
② transferring a solution of an IL-2 protein of formula I to a preparation phase;
③ adding the excipient mother liquor;
④ adding the buffer to adjust the protein concentration;
⑤ sterilizing and pre-filtering;
⑥ sterilizing and filtering to a stock solution bag;
⑦ filling and half-stoppering;
⑧ lyophilizing;
⑨ stoppering, discharging and capping.

In some embodiments, the filling quantity of the lyophilized preparation before lyophilization is about 1.10 to about 1.20 mL, e.g., about 1.15 mL.

In some embodiments, the lyophilized preparation is stored in a dark place at 2-8 °C and is stable for at least 1 month, at least 3 months, at least 6 months, at least 12 months, at least 18 months, at least 24 months, or at least 30 months.

In some embodiments, the lyophilized preparation is stable at 25 °C for at least 1 month, at least 3 months, at least 6 months, or at least 12 months.

In some embodiments, the lyophilized preparation is stable at 40 °C for at least 7 days, at least 14 days, or at least 30 days.

The present disclosure also provides a reconstituted solution comprising IL-2, which is prepared by reconstituting the aforementioned lyophilized preparation.

The present disclosure also provides a method for preparing the reconstituted solution described above, which comprises the step of reconstituting the aforementioned lyophilized preparation, wherein the solution used for reconstitution is selected from, but is not limited to, the group consisting of water for injection, normal saline and a glucose solution such as 5% glucose injection or glucose-sodium chloride injection. The present disclosure also provides an article of manufacture, which comprises a container containing the aforementioned pharmaceutical composition, lyophilized preparation or reconstituted solution. The labeled quantity of the container is adjusted as needed and may be selected from the group consisting of 0.5 mL, 0.6 mL, 0.7 mL, 0.8 mL, 0.9 mL, 1.0 mL, 1.1 mL, 1.2 mL, 1.3 mL, 1.4 mL, 1.5 mL, 1.6 mL, 1.7 mL, 1.8 mL, 1.9 mL, 2.0 mL, 2.1 mL, 2.2 mL, 2.3 mL, 2.4 mL, 2.5 mL, 5.0 mL, and 10 mL. Part of an injection may remain in the container and be lost during clinical administration. Therefore, to ensure the labeled quantity, the filling quantity of the product of the present disclosure should be slightly increased in practical production according to the general requirements for injections in Chinese Pharmacopoeia, Volume IV, 2020 Edition. For about 1 mL of the pharmaceutical composition, the target quantity is set to about 1.15 mL in practical filling production. The quantity range is controlled to be from about 1.10 to about 1.20 mL.

In some embodiments, the inner packaging materials for the container are a tubular injection vial made of neutral borosilicate glass, a butyl bromide rubber stopper for lyophilization and injection, and an aluminum-plastic composite cap for an antibiotic vial.

### Preparation Method

Common methods in the art may be used to prepare the pharmaceutical composition of the present disclosure, including the step of mixing a proper amount of IL-2 with the aforementioned pharmaceutically acceptable excipients.

In some embodiments, after IL-2 is prepared, a pharmaceutical preparation comprising same is prepared, the preparation comprising adding a proper amount of trehalose, mannitol, and other alternative excipient ingredients to the pharmaceutical preparation.

### Use

The present disclosure provides pharmaceutical use of the pharmaceutical composition comprising IL-2, the lyophilized preparation or the reconstituted solution described above for treating an autoimmune disease or alleviating/treating/preventing autoimmune responses following organ transplantation. The autoimmune disease may be selected from the group consisting of the following or any of the following: type I diabetes mellitus, rheumatoid arthritis, multiple sclerosis, chronic gastritis, Crohn's disease, Basedow disease, Bechterew disease, psoriasis, myasthenia gravis, autoimmune hepatitis, APECED, Chrug-Strauss syndrome, ulcerative colitis, glomerulonephritis, Guillain-Barrésyndrome, Hashimoto thyroiditis, lichen sclerosus, systemic lupus erythematodes, PANDAS, rheumatic fever, sarcoidosis, Sjögren's syndrome, Stiff-Man syndrome, scleroderma, Wegener's granulomatosis, vitiligo, autoimmune enteropathy, Goodpasturesyndrome, dermatomyositis, polymyositis, autoimmune allergy, and asthma.

In some embodiments, the IL-2 variant or the derivative thereof may be used in conjunction with an immunosuppressive agent. In some embodiments, the immunosuppressive agent is selected from the group consisting of the following: glucocorticoids, including decortin and prednisol; azathioprine; cyclosporin A; mycophenolatemofetil; tacrolimus; anti-T lymphocyte globulin and anti-CD3 antibodies, including muromonab; anti-CD25 antibodies, including basiliximab and daclizumab; anti-TNF-α antibodies, including infliximab and adalimumab; azathioprine; methotrexate; cyclosporin; sirolimus; everolimus; fingolimod; CellCept; myfortic; and cyclophosphamide.

In some embodiments, the pharmaceutical composition, lyophilized preparation or reconstituted solution of the present disclosure cannot provide a cure but only a partial benefit. In some embodiments, physiological changes with some benefits are also considered therapeutically beneficial. Therefore, in some embodiments, the amount of the IL-2 variant or the derivative or immunoconjugate thereof that provides the physiological changes is considered an "effective amount" or "therapeutically effective amount". The subject, patient or individual in need of treatment is typically a mammal, more particularly a human.

The pharmaceutical composition, lyophilized preparation or reconstituted solution may be administered by any effective route, e.g., by injection, which may be in a form suitable for injection, e.g., by bolus injection or continuous infusion, into the bloodstream of a patient, or intravenous injection, subcutaneous injection, intradermal injection, intraperitoneal injection, etc. Alternatively, it may be administered locally at the tumor site (intratumoral or peritumoral administration). In the case of intratumoral or peritumoral administration, it may be administered via any route, e.g., by topical administration, regional administration, local administration, systemic administration, convection-enhanced delivery, or a combination thereof.

The forms of injection include sterile aqueous solutions or dispersion systems. Further, the pharmaceutical composition described above may be prepared in the form of a sterile powder for instant preparation of sterile injections or dispersions. In any case, the final form of injection must be sterile and must be readily flowable for ease of injection. Furthermore, the pharmaceutical composition must be stable during preparation and storage. Therefore, preferably, the pharmaceutical composition is to be preserved against microbial contamination, such as bacterial and fungal contamination.

### Detailed Description of the Invention

### Terms

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. The disclosure of the published patent document WO2020/125743A1 is incorporated herein in its entirety.

The three-letter and single-letter codes for amino acids used in the present application are as described in J. Biol. Chem., 243, p3558 (1968).

"Interleukin-2" or "IL-2" refers to any natural IL-2 of any vertebrate source, including mammals such as primates (e.g., humans) and rodents (e.g., mice and rats). The term encompasses unprocessed IL-2 as well as any form of processed IL-2 derived from cells. The term also encompasses naturally occurring IL-2 variants, such as splice variants or allelic variants. An exemplary amino acid sequence of wild-type human IL-2 is set forth in SEQ ID NO: 1. Unprocessed human IL-2 additionally comprises an N-terminal signal peptide of 20 amino acids (set forth in SEQ ID NO: 272 in WO2012107417), which is absent in the mature IL-2 molecule.

"Amino acid mutations" include amino acid substitutions, deletions, insertions, modifications, and any combination thereof, to obtain a final construct that possesses desired properties, such as enhanced stability. Amino acid sequence deletions and insertions include amino-terminal and/or carboxyl-terminal deletions and amino acid insertions. An example of terminal deletion is the deletion of an alanine residue at position 1 of full-length human IL-2. Preferred amino acid mutations are amino acid substitutions. To alter the binding properties of, for example, an IL-2 polypeptide, non-conservative amino acid substitutions may be made, i.e., one amino acid is replaced with another amino acid having different structural and/or chemical properties. Preferred amino acid substitutions include the replacement of hydrophobic amino acids with hydrophilic amino acids. Amino acid substitutions include the replacement with non-naturally occurring amino acids or with naturally occurring amino acid derivatives of the 20 standard amino acids (e.g., 4-hydroxyproline, 3-methylhistidine, ornithine, homoserine and 5-hydroxylysine). Amino acid mutations can be generated using genetic or chemical methods well known in the art, including site-directed mutagenesis, PCR, gene synthesis, chemical modification, and the like.

"Wild-type IL-2" is a form of IL-2 that has a wild-type amino acid at each amino acid position of a variant IL-2 but otherwise is identical to the variant IL-2 polypeptide. For example, if the IL-2 variant is full-length IL-2 (i.e., an IL-2 that is not fused or conjugated to any other molecule), then the corresponding wild-type form of the variant is full-length natural IL-2. If the IL-2 variant is a fusion of IL-2 and another polypeptide encoded downstream thereof (e.g., an antibody chain), then the corresponding wild-type form of the IL-2 variant is an IL-2 of a wild-type amino acid sequence that is fused to the same downstream polypeptide. Furthermore, if the IL-2 variant is a truncated form of IL-2 (a mutated or modified sequence within the non-truncated portion of IL-2), then the wild-type form of the IL-2 variant is a similarly truncated IL-2 with a wild-type sequence. To compare the IL-2 receptor binding affinities or biological activities of various forms of IL-2 variants and their corresponding wild-type forms of IL-2, the term "wild-type" encompasses forms of IL-2 that contain one or more amino acid mutations that do not affect IL-2 receptor binding, e.g., the substitution of cysteine at the position corresponding to residue 125 of human IL-2 with alanine (C125A), compared to naturally occurring, natural IL-2. In some embodiments, the wild-type IL-2 comprises an amino acid sequence set forth in SEQ ID NO: 1.

"Derivative" is intended to be interpreted in its broad sense and includes any IL-2-related product. The IL-2 variant or derivative includes, but is not limited to, human and non-human IL-2 homologs, fragments or truncations, fusion proteins (e.g., fused to a signal peptide or other active components (e.g., an antibody or antigen-binding fragment thereof) and inactive components), modified forms (e.g., PEGylated, glycosylated, albumin conjugated/fused, Fc conjugated and/or fused, and hydroxyethylated forms), conservatively modified proteins, etc.

"High-affinity IL-2 receptor" refers to the heterotrimer form of the IL-2 receptor that consists of a receptor γ subunit (also known as a common cytokine-receptor γ subunit, γc, or CD132), a receptor β subunit (also known as CD122 or p70), and a receptor α subunit (also known as CD25 or p55). In contrast, "moderate-affinity IL-2 receptor" refers to an IL-2 receptor that comprises only γ and β subunits but no α subunit (see, e.g., Olejniczak and Kasprzak, MedSci Monit 14, RA179-189 (2008)).

"Regulatory T cell", "T regulatory cell" or "Treg" refers to a specialized CD4+ T cell type that can inhibit the response of other T cells. Tregs are characterized by expression of the α subunit of IL-2 receptor (CD25) and the transcription factor forkhead box P3 (FOXP3), and play a key role in the induction and maintenance of peripheral self-tolerance to antigens, including those expressed by tumors. Treg requires IL-2 for its function and development as well as for the induction of its inhibitory characteristics. "Effector cell" refers to the lymphocyte population that mediates the cytotoxic effect of IL-2. Effector cells include effector T cells such as CD8+ cytotoxic T cells, NK cells, lymphokine-activated killer (LAK) cells, and macrophages/monocytes.

"Conservative modifications" are applicable to amino acid and nucleotide sequences. For particular nucleotide sequences, conservative modifications refer to those nucleic acids encoding identical or substantially identical amino acid sequences, or, in the case of nucleotides not encoding amino acid sequences, to substantially identical nucleotide sequences. For amino acid sequences, "conservative modifications" refer to the replacement of amino acids in a protein with other amino acids having similar characteristics (e.g., charge, side chain size, hydrophobicity/hydrophilicity, backbone conformation, and rigidity) such that changes can be made frequently without altering the biological activity of the protein. Those skilled in the art recognize that, generally speaking, a single amino acid replacement in a non-essential region of a polypeptide does not substantially change the biological activity (see, e.g., Watson et al. (1987) Molecµlar Biology of the Gene, The Benjamin/Cummings Pub. Co., p224, (4th edition)).

"PEGylated" refers to linking of at least one PEG molecule to another molecule (e.g., a therapeutic protein). For example, Adagen (a PEGylated preparation of adenosine deaminase) is approved for the treatment of severe combined immunodeficiency disease. It has been shown that the linking of polyethylene glycol can prevent proteolysis (see, e.g., Sada et al (1991) J. Fermentation Bioengineering 71: 137-139). In the most common form, PEG is a linear or branched polyether linked at one end to a hydroxy group and has the following general structure: HO-(CH2CH2O)n-CH2CH2-OH. To couple PEG to a molecule (polypeptides, polysaccharides, polynucleotides and small organic molecules), the PEG can be activated by preparing a derivative of the PEG having a functional group at some or both terminals. A common route for PEG conjugation of proteins is to activate the PEG with functional groups suitable for reaction with lysine and N-terminal amino acid groups. In particular, common reactive groups involved in conjugation are the α or ε amino groups of lysine. The reaction of a pegylation linking group with a protein leads to the attachment of the PEG moiety predominantly at the following sites: the α amino group at the N-terminal of the protein, the ε amino group on the side chain of lysine residues, and the imidazole group on the side chain of histidine residues. Since most recombinant proteins possess a single α and a number of ε amino and imidazole groups, numerous positional isomers can be generated depending on the chemical properties of linking groups.

"Administering", "giving" and "treating", when applied to animals, humans, experimental subjects, cells, tissues, organs or biological fluid, refer to contacting an exogenous drug, a therapeutic agent, a diagnostic agent or composition with the animals, humans, subjects, cells, tissues, organs or biological fluid. "Administering", "giving" and "treating" can refer to, for example, therapeutic, pharmacokinetic, diagnostic, research and experimental methods. The treatment of cells comprises making the reagent in contact with the cells and making the reagent in contact with fluid, where the fluid is in contact with the cells. "Administering", "giving" and "treating" also refer to treating, e.g., cells by reagents, diagnosis, binding compositions or by another cell *in vitro* and *ex vivo.* "Administering" and "treating", when applied to humans, veterinary or research subjects, refer to therapeutic treatment, preventive or prophylactic measures, and research and diagnostic applications.

"Treatment" refers to administering a therapeutic agent, such as any one of the IL-2 variants and derivatives thereof of the present disclosure or a composition comprising the variant or derivative, either internally or externally to a subject who has one or more symptoms of a disease, or one or more suspected symptoms of a disease, or is susceptible to one or more symptoms of a disease, and the therapeutic agent is known to have a therapeutic effect on these symptoms. Generally, the therapeutic agent is administered in an amount effective to alleviate one or more symptoms of the disease in the subject or population being treated, whether by inducing regression of such symptoms or inhibiting such symptoms from progressing to any clinically unmeasurable degree.

"Effective amount" comprises an amount sufficient to ameliorate or prevent a symptom or sign of a medical condition. An effective amount also refers to an amount sufficient to allow or facilitate diagnosis. The effective amount for a particular subject or veterinary subject may vary depending on the factors such as the disorder to be treated, the general health of the subject, the method and route and dosage of administration, and the severity of side effects. An effective amount may be the maximum dose or administration regimen to avoid significant side effects or toxic effects.

"Buffer" refers to a buffer that resists changes in pH by the action of its acid-base conjugate components. Examples of a buffer that controls the pH in an appropriate range include acetate, succinate, gluconate, histidine salt, oxalate, lactate, phosphate, citrate, tartrate, fumarate, glycylglycine and other organic acid buffers.

"Histidine salt buffer" is a buffer containing histidine ions. Examples of the histidine salt buffer include, but are not limited to, histidine-hydrochloric acid buffer, histidine-acetate buffer, histidine-phosphate buffer, histidine-sulfate buffer, and the like. Histidine-acetate buffer or histidine-hydrochloric acid buffer is preferred. Histidine-acetate buffer is prepared from histidine and acetic acid. Histidine-hydrochloric acid buffer is prepared from histidine and hydrochloric acid.

"Citrate buffer" is a buffer containing citrate ions. Examples of the citrate buffer include citric acid-sodium citrate, citric acid-potassium citrate, citric acid-calcium citrate, citric acid-magnesium citrate, and the like. Preferably, the citrate buffer is citric acid-sodium citrate.

"Succinate buffer" is a buffer containing succinate ions. Examples of the succinate buffer include succinic acid-sodium succinate, succinic acid-potassium succinate, succinic acid-calcium succinate, and the like. Preferably, the succinate buffer is succinic acid-sodium succinate.

"Phosphate buffer" is a buffer containing phosphate ions and is selected from any phosphate buffer known to those skilled in the art and suitable for use in the system of the present disclosure. The phosphate buffer component is preferably a phosphate buffer comprising one or more phosphates, for example, a mixture of a monobasic phosphate, a dibasic phosphate, and the like. Particularly useful phosphate buffers are selected from the group consisting of alkali metal and/or alkaline earth metal phosphates. Examples of the phosphate buffer include disodium hydrogen phosphate-sodium dihydrogen phosphate, disodium hydrogen phosphate-potassium dihydrogen phosphate, disodium hydrogen phosphate-citric acid, Tris-HCl buffer, sodium phosphate-phosphoric acid solution, disodium hydrogen phosphate-phosphoric acid solution, sodium phosphate-sodium dihydrogen phosphate solution, and the like. Preferably, the phosphate buffer is disodium hydrogen phosphate-sodium dihydrogen phosphate. Sodium dihydrogen phosphate and disodium hydrogen phosphate may be anhydrous or hydrated as needed, e.g., anhydrous disodium hydrogen phosphate, sodium dihydrogen phosphate monohydrate, sodium dihydrogen phosphate dihydrate, disodium hydrogen phosphate heptahydrate, or disodium hydrogen phosphate dodecahydrate.

"Acetate buffer" is a buffer containing acetate ions. Examples of the acetate buffer include acetic acid-sodium acetate, acetic acid-histidine salt, acetic acid-potassium acetate, acetic acid-calcium acetate, acetic acid-magnesium acetate, and the like. Preferably, the acetate buffer is acetic acid-sodium acetate.

The term "pharmaceutical composition" refers to a mixture containing one or more of the compounds or the physiologically/pharmaceutically acceptable salts or prodrugs thereof described herein, and other chemical components, for example, physiologically/pharmaceutically acceptable carriers and excipients. The purpose of the pharmaceutical composition is to maintain the stability of the active ingredient of the antibody and promote the administration to an organism, which facilitates the absorption of the active ingredient, thereby exerting biological activity.

As used herein, "pharmaceutical composition" and "preparation" are not mutually exclusive.

"Lyophilized preparation" refers to a preparation or a pharmaceutical composition obtained by freeze-drying a pharmaceutical composition or a preparation in liquid or solution form in vacuum.

The terms "about" and "approximately" as used herein mean that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1. Alternatively, "about" or "comprising essentially" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. When a particular value is provided in the present application and claims, unless otherwise stated, the meaning of "about" or "comprising essentially" should be assumed to be within an acceptable error range for that particular value.

The lyophilized preparation of the present disclosure can achieve a stable effect: a pharmaceutical composition in which the protein substantially retains its physical stability, chemical stability and/or biological activity after storage. The storage period is generally selected based on a predetermined shelf life of the pharmaceutical composition. There are a variety of analytical techniques currently available for determining protein stability, including RP-HPLC, SE-HPLC and IE-HPLC. The stability after storage for a selected period of time at a selected temperature can be determined. Typical acceptable criteria for stability are as follows: typically, no more than about 10%, e.g., no more than about 5%, of protein (e.g., an IL-2 variant or derivative) aggregates or degrades, as measured by RP-HPLC, SE-HPLC or IE-HPLC. The preparation is a pale yellow, nearly colorless and clear liquid, or colorless, or clear to slightly opalescent, by visual analysis. The concentration, pH and osmolality of the preparation have changes of no more than ±10%. Typically, reductions of no more than about 10%, e.g., no more than about 5%, are observed. A protein "retains its physical stability" in a pharmaceutical preparation if it shows no significant increase in aggregation, precipitation and/or denaturation upon visual inspection of color and/or clarity, or as determined by UV light scattering, size exclusion chromatography (SEC) and dynamic light scattering (DLS). Changes in protein conformation can be assessed by fluorescence spectroscopy (which determines the protein tertiary structure) and by FTIR spectroscopy (which determines the protein secondary structure).

An IL-2 variant or derivative "retains its chemical stability" in a pharmaceutical preparation if it shows no significant chemical change. Chemical stability can be assessed by detecting and quantifying chemically changed forms of the protein. Degradation processes that often change the chemical structure of proteins include hydrolysis or truncation (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

An IL-2 variant or derivative "retains its biological activity" in a pharmaceutical preparation if the biological activity of the antibody at a given time is within a predetermined range of the biological activity exhibited during the preparation of the pharmaceutical preparation. The biological activity of IL-2 can be determined, for example, through cell or downstream signaling pathway activation tests.

In the present disclosure, "IL-2 variant" and "IL-2 analog" are used interchangeably. "Relative molecular weight" of PEG in the present disclosure refers to the relative molecular mass determined by MALDI-TOF. The number of PEG repeat units can be calculated using the relative molecular weight of PEG.

### DETAILED DESCRIPTION

The present disclosure is further described below with reference to examples, which, however, are not intended to limit the protection scope of the present disclosure.

Experimental procedures without specific conditions indicated in the examples or test examples are generally conducted according to conventional conditions, or according to conditions recommended by the manufacturers of the starting materials or commercial products, See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY; or alternatively, the conditions recommended by the manufacturers of the starting materials or commercial products are used. Reagents without specific origins indicated were commercially available conventional reagents.

### Example 1. Stability Study of IL-2 as Drug Substance

IL-2 is a PEGylated recombinant human interleukin-2 analog of the structure shown below (formula I). 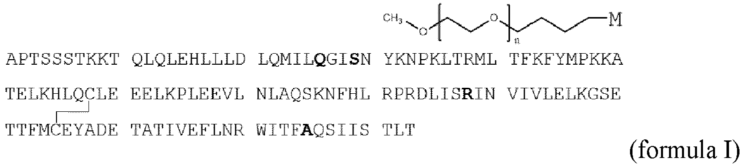 (Note: The positions in bold are positions of mutations relative to the wild type: N26Q, N29S, N88R, C125A. The linked Cys58-Cys105 indicates the position of a disulfide bond.)

The structure of formula I is prepared from an IL-2 variant set forth in SEQ ID NO: 2 and a PEG molecule with a molecular weight of about 20 KD.

Relative molecular weight of formula I: about 36 kD.

Appearance: a colorless or yellowish clear liquid.

pH: 4.5-6.0.

The results of accelerated testing show that the quality of the product did not significantly change in one month under an accelerated condition. Three batches of bulk solutions of the drug substance (the solution system of the bulk solutions was the same as the solution system obtained by screening in later examples) were left to stand under an accelerated condition (5 °C ± 3 °C) for one month, during which there was no significant change in each of the indicators, and all the results were within the ranges of quality standards. The solution system of the IL-2 bulk solutions comprises 4 ± 0.5 g/L drug substance and 10 mM histidine-hydrochloric acid buffer having a pH of 5.30 ± 0.05.

The molecular weight is 35.5 ± 3.6 KD, as determined by SDS-polyacrylamide gel electrophoresis (reducing) in the present disclosure.

The microorganism and bacterial endotoxin levels of the drug substance will affect the quality of finished preparation products, so the microorganism and bacterial endotoxin levels of the drug substance need to be controlled to conform with the general requirements in Chinese Pharmacopoeia, Volume IV, 2015 Edition.

### Example 2. Buffer System and pH Screening for IL-2 Preparation

To screen for a suitable buffer system, solutions with 10 mM citric acid-sodium hydroxide (pH 4.0-5.5), 10 mM succinic acid-sodium hydroxide (pH 5.0), 10 mM histidine-hydrochloric acid (pH 5.5 and 6.0) and 10 mM phosphate (pH 6.0-8.0) as buffer systems were prepared while maintaining the IL-2 variant (represented by formula I) at a concentration of 2 mg/mL, using 5% mannitol as an excipient, and using 0.05 mg/mL polysorbate 80 as a stabilizer. The preparations with the different buffer systems described above were then left to stand at 40 °C for 14 d, and the related indicators were measured at 0 d, 7 d, and 14 d. The results for the buffer system samples are shown in Table 2.

**Table 2. The results of the 40 °C, 14-d investigation of preparations of the IL-2 variant (represented by formula I) with different buffer systems**

| Buffer system | pH | RP-HPLC | | | SE-HPLC | | |
|---|---|---|---|---|---|---|---|
| | | 0d | 7d | 14d | 0d | 7d | 14d |
| 10 mM citric acid-sodium hydroxide | 4.0 | 98.8% | 97.1% | / | 97.7% | 70.6% | / |
| | 4.5 | 98.7% | 97.1% | / | 99.4% | 58.6% | / |
| | 5.0 | 94.7% | 59.6% | | 99.2% | 34.9% | |
| | 5.5 | 98.8% | 95.0% | / | 99.3% | 79.6% | / |
| 10 mM succinic acid-sodium hydroxide | 5.0 | 98.4% | 93.3% | / | 99.5% | 93.3% | / |
| 10 mM histidine-hydrochloric acid | 5.5 | 98.8% | 96.9% | 96.6% | 99.5% | 97.7% | 96.6% |
| | 6.0 | 98.8% | 95.3% | 92.9% | 99.4% | 95.9% | 91.3% |
| 10 mM phosphate | 6.0 | 98.8% | 94.1% | 90.8% | 99.3% | 88.2% | 81.4% |
| | 6.5 | 98.8% | 91.5% | 86.6% | 99.2% | 77.2% | 67.8% |
| | 7.0 | 98.8% | 89.5% | 86.8% | 99.2% | 72.1% | 59.7% |
| | 7.5 | 98.7% | 87.8% | / | 99.2% | 66.6% | / |
| | 8.0 | 98.7% | 85.5% | / | 99.2% | 62.4% | / |

| | | | | | | | |
|---|---|---|---|---|---|---|---|
| (Note: In the table, "/" or a blank indicates no measurement.) | | | | | | | |

From the results in Table 2, it can be seen that the SEC-HPLC and RP-HPLC data show that the histidine-hydrochloric acid buffer systems (pH 5.5 and 6.0) are relatively stable, and that succinate, citrate and phosphate systems all exhibited varying degrees of degradation. The histidine-hydrochloric acid buffer 5.5 system is the best. Therefore, the histidine-hydrochloric acid buffer system was selected.

### Example 3. Lyoprotectant and pH Screening for IL-2 Preparation

It can be seen from Example 2 that the histidine-hydrochloric acid buffer systems have good stability and pH 5.5 is better than pH 6.0. It was speculated that low pH is good for protein stabilization, so histidine pH 5.0 was added on the basis of pH 5.5 and 6.0. In the phosphate system, in solution states, the pH 6.0 and 7.0 groups have higher protein stability than other pH groups of the same system. In this example, the effect of the 2 pH systems on protein stability was investigated.

5% mannitol, 1% mannitol + 7% sucrose, and 1% mannitol + 7% trehalose were used as excipients to investigate their protective effects on IL-2 protein while maintaining the IL-2 variant (represented by formula I) at a concentration of 2 mg/mL and using 0.05 mg/mL polysorbate 80 as a stabilizer. The preparations of the different formulations described above were left to stand at 40 °C for 30 d, and the related indicators were measured at 0 d, 7 d, 14 d, and 30 d. The results are shown in Table 3.

**Table 3. The results of the 40 °C, 30-d investigation of preparations of the IL-2 variant (represented by formula I) with different pH and lyoprotectants**

| Buffer system | Protectant | Appearance | RP-HPLC (%) | | | | SE-HPLC (%) | | | |
|---|---|---|---|---|---|---|---|---|---|---|
| | | | 0d | 7d | 14d | 30d | 0d | 7d | 14d | 30d |
| pH 5.0 histidine-hydrochloric acid | 5% mannitol | White porous solid | 98. 0 | 98. 3 | 98. 7 | 96. 7 | 99. 6 | 99. 6 | 99. 1 | 99. 4 |
| pH 5.5 histidine-hydrochloric acid | 5% mannitol | White porous solid | 98. 0 | 98. 6 | 98. 6 | 97. 0 | 99. 4 | 98. 8 | 98. 1 | 98. 7 |
| pH 6.0 histidine-hydrochloric acid | 5% mannitol | White porous solid | 97. 5 | 98. 7 | 98. 7 | 96. 2 | 99. 4 | 98. 1 | 97. 6 | 97. 5 |
| pH 6.0 phosphate | 5% mannitol | White porous solid | 97. 9 | 98. 5 | 98. 4 | 97. 3 | 99. 4 | 98. 5 | 98. 0 | 98. 0 |
| pH 7.0 phosphate | | | 97. 9 | 96. 6 | 98. 4 | 91. 3 | 99. 0 | 96. 1 | 95. 6 | 93. 2 |
| pH 5.5 histidine-hydrochloric acid | 7% sucrose + 1% mannitol | White porous solid Significant bottom contraction | 97. 7 | 98. 4 | 98. 6 | 96. 9 | 99. 4 | 99. 4 | 99. 5 | 99. 6 |
| pH 5.5 histidine-hydrochloric acid | 7% trehalose + 1% mannitol | White porous solid Slight bottom contraction | 97. 7 | 98. 2 | 98. 5 | 97. 1 | 99. 6 | 99. 1 | 99. 1 | 99. 5 |

As can be seen from the above results, the 40 °C, 0-d, 7-d, 14-d and 30-d experiments show that the phosphate pH 7.0 system has poor RP and SE results, and the phosphate pH 6.0 system was less stable in solution than the histidine-hydrochloric acid system in the early stage and was at the limit of the buffer capacity, so the phosphate system was excluded; in the cases of histidine-hydrochloric acid pH 5.5, both 7% sucrose + 1% mannitol and 7% trehalose + 1% mannitol have better 30-d SE results than 5% mannitol; the histidine-hydrochloric acid pH 6.0 system has lower 30-d RP and SE values than the histidine-hydrochloric acid pH 5.5 and 5.0 systems, indicating poor stability. Therefore, the determined suitable pH range is 5.0-5.5. By way of example, 5.3 was selected as the target pH.

### Example 4. Excipient Proportion and Polysorbate 80 Concentration Screening for IL-2 Preparation

Different proportions of the excipient will not only affect the appearance of the product but also the stability of the protein. Mannitol and trehalose were preliminarily selected as excipients and the amount of the excipients was investigated. Specifically, 1% mannitol + 7% trehalose and 3% mannitol + 4% trehalose samples were prepared while using pH 5.3 10 mM histidine-hydrochloric acid as a buffer system and maintaining the IL-2 variant (represented by formula I) at a concentration of 2 mg/mL, and meanwhile, the effect of different concentrations of polysorbate 80 on protein stability was investigated under the 3% mannitol + 4% trehalose condition. The samples of the different formulations described above were then left to stand at 40 °C for 30 d, and the related indicators were measured at 0 d, 14 d, and 30 d. The results for the IL-2 variant (represented by formula I) are shown in Table 4.

**Table 4. The results of the 40 °C, 30-d investigation of preparations of the IL-2 variant (represented by formula I) with different excipient proportions and polysorbate 80 concentrations**

| Test | Excipient | Appearance | Polysorbate concentration (mg/mL) | 0d | 14d | 30d |
|---|---|---|---|---|---|---|
| RP-HPLC | 1% mannitol + 7% trehalose | White porous solid | 0.05 | 98.2 | 98.3 | 97.9 |
| | | Slight bottom contraction | | | | |
| | 3% mannitol + 4% trehalose | White porous solid | 0.05 | 98.2 | 98.3 | 97.9 |
| | | White porous solid | 0.02 | 98.2 | 98.4 | 96.1 |
| | | White porous solid | 0.1 | 98.0 | 98.3 | 98.2 |
| SEC-HPLC | 1% mannitol + 7% trehalose | White porous solid | 0.05 | 99.9 | 99.8 | 99.5 |
| | | Slight bottom contraction | | | | |
| | 3% mannitol + 4% trehalose | White porous solid | 0.05 | 99.9 | 99.8 | 99.5 |
| | | White porous solid | 0.02 | 99.9 | 99.8 | 99.0 |
| | | White porous solid | 0.1 | 99.9 | 99.8 | 99.6 |

As can be seen from the results, after storage at a high temperature of 40 °C for 30 d, in the cases of 0.05 mg/mL polysorbate, the 1% mannitol + 7% trehalose and 3% mannitol + 4% trehalose formulations are almost the same in stability; in the cases of 3% mannitol + 4% trehalose, when polysorbate 80 is at 0.05 mg/mL and 0.1 mg/mL, there is no significant difference in stability, but considering safety, it is advisable to use as little polysorbate 80 as possible. Therefore, 0.05 mg/mL polysorbate 80 was selected. Considering the stabilities and appearances of the samples, the amount of the excipient composition in the preparation of the IL-2 variant (represented by formula I) was determined to be 3% mannitol + 4% trehalose-that is, the concentration of mannitol is 30 mg/mL, the concentration of trehalose is 40 mg/mL, and the concentration of polysorbate is 0.05 mg/mL.

In the present disclosure, the molecular formula of trehalose is C₁₂H₂₂O₁₁·2H₂O; the molecular formula of histidine is C₆H₉N₃O₂; the molecular formula of histidine-hydrochloric acid is C₆H₉N₃O₂.HCl.H₂O; the water for injection was removed in the lyophilization process; when the labeled quantity of the product is 1 mL, the target quantity can be set to 1.15 mL in practical production.

Although the foregoing invention has been described in detail by way of drawings and examples for purposes of clarity of understanding, the description and examples should not be construed as limiting the scope of the present disclosure. The disclosures of all patents and scientific literature cited herein are clearly incorporated by reference in their entirety.

## Claims

1. A pharmaceutical composition, comprising IL-2, mannitol, trehalose and a histidine salt buffer, wherein mannitol and trehalose are in a mass ratio of 1:7 to 3:4, preferably 3:4.

2. The pharmaceutical composition according to claim 1, wherein the IL-2 is an IL-2 variant or a derivative thereof;
the IL-2 variant or the derivative thereof contains mutations N26Q and N29S, and preferably further contains a mutation selected from the group consisting of N88R, N88G, N88I and N88D.

3. The pharmaceutical composition according to any one of the preceding claims, wherein the mannitol is at a concentration of about 10 mg/mL to about 100 mg/mL, preferably about 10 mg/mL to about 50 mg/mL, and more preferably about 30 mg/mL.

4. The pharmaceutical composition according to any one of the preceding claims, wherein the trehalose is at a concentration of about 10 mg/mL to about 100 mg/mL, preferably about 30 mg/mL to about 70 mg/mL, and more preferably about 40 mg/mL.

5. The pharmaceutical composition according to any one of the preceding claims, wherein the IL-2 is at a concentration of about 0.1 mg/mL to about 100 mg/mL, preferably about 1 mg/mL to about 10 mg/mL, and more preferably about 2 mg/mL.

6. The pharmaceutical composition according to any one of the preceding claims, further comprising a surfactant, wherein the surfactant is preferably a polysorbate, and more preferably polysorbate 80.

7. The pharmaceutical composition according to claim 6, wherein the surfactant is at a concentration of about 0.01 mg/mL to about 0.2 mg/mL, preferably about 0.05 mg/mL to about 0.1 mg/mL, and more preferably about 0.05 mg/mL.

8. The pharmaceutical composition according to any one of the preceding claims, wherein the histidine salt buffer has a concentration of about 2 mM to about 50 mM, preferably about 5 mM to about 20 mM, and more preferably about 10 mM.

9. The pharmaceutical composition according to any one of the preceding claims, wherein the pharmaceutical composition has a pH of about 4.5 to about 6.0, preferably about 5.0 to about 5.6, and more preferably about 5.3.

10. The pharmaceutical composition according to any one of the preceding claims, wherein the pharmaceutical composition comprises:
(a) about 0.1 mg/mL to about 100 mg/mL IL-2,
(b) about 10 mg/mL to about 100 mg/mL mannitol,
(c) about 10 mg/mL to about 100 mg/mL trehalose,
(d) about 0.01 mg/mL to about 0.2 mg/mL polysorbate, and
(e) about 2 mM to about 50 mM histidine salt buffer,
the pharmaceutical composition having a pH of about 4.5 to about 6.0;
preferably, the pharmaceutical composition comprises:
(a) about 1 mg/mL to about 10 mg/mL IL-2,
(b) about 10 mg/mL to about 50 mg/mL mannitol,
(c) about 30 mg/mL to about 70 mg/mL trehalose,
(d) about 0.02 mg/mL to about 0.1 mg/mL polysorbate 80, and
(e) about 5 mM to about 20 mM histidine salt buffer,
the pharmaceutical composition having a pH of about 5.0 to about 5.5;
more preferably, the pharmaceutical composition comprises:
(a) about 2 mg/mL IL-2,
(b) about 30 mg/mL mannitol,
(c) about 40 mg/mL trehalose,
(d) about 0.05 mg/mL polysorbate 80, and
(e) about 10 mM histidine salt buffer,
the pharmaceutical composition having a pH of about 5.3.

11. The pharmaceutical composition according to any one of the preceding claims, wherein the pharmaceutical composition further comprises a physiologically acceptable solvent, preferably normal saline, water for injection or a glucose solution.

12. The pharmaceutical composition according to any one of the preceding claims, wherein the IL-2 is an IL-2 variant or a derivative thereof and comprises an amino acid sequence selected from any one of SEQ ID NOs: 2 and 8-12, and methionine (M) at position 1 of the SEQ ID NOs: 2 and 8-12 may be present or absent.

13. The pharmaceutical composition according to any one of the preceding claims, wherein the IL-2 is monomeric, and/or PEGylated, and/or glycosylated, and/or albumin-conjugated or -fused, and/or Fc-fused, and/or hydroxyethylated, and/or de-O-glycosylated;
preferably, PEG is linked to the N-terminus of the IL-2 variant;
more preferably, PEG has a relative molecular weight of about 5 KD to about 50 KD;
most preferably, PEG has a relative molecular weight of about 20 KD.

14. The pharmaceutical composition according to any one of the preceding claims, wherein the IL-2 comprises a structure of formula I, wherein the PEG has a relative molecular weight of about 20 KD; the amino acid sequence is identical to an amino acid sequence set forth in SEQ ID NO: 2,

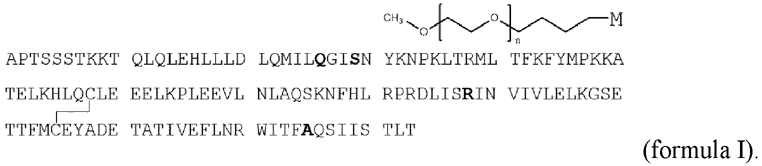

15. A lyophilized preparation, wherein the lyophilized preparation is obtained by lyophilizing the pharmaceutical composition according to any one of claims 1-14, or the lyophilized preparation can be reconstituted to form the pharmaceutical composition according to any one of claims 1-14.

16. A reconstituted solution, wherein the reconstituted solution is prepared by reconstituting the lyophilized preparation according to claim 15.

17. Use of the pharmaceutical composition according to any one of claims 1-14, the lyophilized preparation according to claim 15, or the reconstituted solution according to claim 16 in the preparation of a medicament for treating and/or preventing an autoimmune disease or autoimmune responses following organ transplantation, wherein preferably, the autoimmune disease is selected from the group consisting of type I diabetes mellitus, rheumatoid arthritis, multiple sclerosis, systemic lupus erythematodes (SLE), eczema, and asthma.

18. A method for preparing the pharmaceutical composition according to any one of claims 1-14, comprising the step of mixing IL-2 with pharmaceutically acceptable excipients.
